# EUROPEAN PATENT APPLICATION

(11) **EP 1 300 681 A1**
(43) Date of publication of application: **09.04.2003**
(21) Application number: 01949936.7
(22) Date of filing: 11.07.2001
(51) Int. Cl.: G01N 33/50, G01N 33/15, G01N 33/574

(54) **MEANS OF ASSAYING IMMUNE FUNCTION**

(30) Priority: 12.07.2000 JP 2000211742
(71) Applicant: Orient Cancer Therapy Co. Ltd, Mitaka-shi, Tokyo 181-0015 (JP)
(72) Inventor: YAGITA, Akikuni, Mitaka-shi, Tokyo 181-0015 (JP)
(74) Representative: Krauss, Jan
(86) International application number: JP0106007
(87) International publication number: WO02004944

(57) **Abstract**

There is provided a novel means of assaying an immune function. That is a means for assaying an immune function where a CD3CD161/Vα24Vβ11 ratio is used as an index.

## Description

This application claims priority from Japanese Patent Application No.2000-211742, which is incorporated herein by reference.

### Technical Field

The present invention relates to a novel means of assaying an immune function paying attention to activation of NKT cells.

### Background of the Invention

Yagita, M. D. who is an inventor of the present application has previously paid his attention to the usefulness of a substance which induces interleukin 12 (IL-12) *in vivo* as an epoch-making means in the therapy of cancer, found that AHCC which is a processed product of mycelia of *Cortinellus shiitake* and established a therapeutic method for cancer which is named a novel immunotherapy. Although IL-12 *per se* has an anticancer effect, there has been a fact that side effect is resulted when IL-12 *per se* is directly administered to living matters and the patient is not durable to the therapy whereby IL-12 *per se* has been unable to be used as an anticancer agent. However, a preparation containing AHCC reported by Yagita has a significant therapeutic and life-prolonging effect in the therapy of cancer. Thus, Yagita has achieved an object of therapy of cancer by administration of an effective amount of AHCC whereby IL-12 can be induced *in vivo* (cf. Japanese Patent Laid-Open Gazette Hei-10/139,670).

IL-12 has a potentiating action for the production of interferon γ (IFNγ) and activating and potentiating effects for killer T cells, LAK cells (lymphokine activated killer cells) and natural killer (NK) cells playing a role of cellular immune in living matter. IFNγ is cytokine whereby immune response of living matter is induced to such a state that T helper 1 cells (Th1) are able to act (the state where NKT cells and killer T cells are apt to achieve their effects or, in other words, the state where interleukin 2 (IL-2) and IL-12 are produced in large amounts). Killer T cells and LAK cells have been known to be the cells which participate in cancer immune. Although it has been reported that NK cells also participate in an anticancer action of living matter, it has been proved by Yagita that, in NK cells, clinical anticancer effect is not correlated to its activity but inducing amount for the production of IL-12 and NK activity are rather in a completely reversed correlation and it has been concluded that NK cells do not participate in an anticancer action in human being.

At present, it has been established by Yagita that a substance having an inducing ability for the production of IL-12 has a possibility of being a hopeful carcinostatic substance.

However, in some of patients suffering from cancer, production of IL-12 is not sufficiently induced even by administration of AHCC and the therapeutic effect is not achieved and, even when production of IL-12 is induced, the therapeutic effect is not sometimes achieved. Therefore, there has been a further demand for developing a novel therapeutic agent for cancer which acts by a different mechanism from the anticancer effect of AHCC.

It has been known that, in an action mechanism of cancer immune, amount of cytokine which is produced or induced *in vivo* is an important element and there have been already attempted and carried out the methods where cancer is treated by administration, induction or production of cytokine which is said to have an anticancer effect. However, although the relations between cancer and immune and between cancer and cytokine have been made clear, curing and life-prolonging effect for cancer thereof has been noted only in 50% or less patients. In recent years, natural killer T (NKT) cells (Cui, J. et al., *Science,* 278, 1623, 1997) have been found as the cells participating in cancer immune. The NKT cell is one of the cells participating in immune system and has functions of a potent cytokine-producing ability and, particularly, an IFNγ-producing ability, a cytotoxic property via Fas and perforin, etc. Accordingly, it is expected that curing and life-prolonging effect for patients suffering from cancer can be further improved when the said cell is activated.

Taniguchi, et al. have found a specific glycolipid antigen recognized by a specific T cell antigen receptor (TCR) which is Vα24Vβ11 owned by the NKT cell and reported that the said antigen is α-galactosylceramide. They have further proved that, in cancer-bearing mice to which α-galactosylceramide is administered, NKT cell is activated and, although disappearance of cancer is not noted, metastasis can be suppressed.

It has been reported that, for NKT cell, there is an NK cell antigen receptor (NKR-P1; natural killer P1) as another receptor (Special Issue for Basis and Clinic of NKT Cells: *Saishin Igaku,* Vol. 55, No. 4, 2000, pages 818∼823). NKR-P1 also participates in activation of NKT cells.

The problem which is to be solved by the present invention is to clarify the activation mechanism of NKT cells and to provide a novel means for assaying immune function. Yagita, D. M. who is an inventor of the present application has already found that the ratio of Th1/Th2 participates in achievement of immune function and that the system where TH1 mainly works in the said ratio is effective in a cancer immune therapy, and the problem to be solved is particularly to provide a novel means for assay of immune function in place of the measure for the above.

### Disclosure of the Invention

In order to solve the above-mentioned problem, the present inventor has carried out repeated investigations for cancer immune cascade in the prevention or the therapy of cancer and found that, in a cascade in which activated NKT cell bearing cancer immune is participated, actions of two different antigen receptors participating in activation of NKT cells or, in other words, NKR-P1 (natural killer receptor-P1) and Vα24Vβ11 are entirely different and that the said relation is able to be a substitute for the ratio of Th1/Th2 whereupon the present invention has been achieved.

Thus, the present invention comprises the followings.
1. A means for assaying an immune function using the ratio of CD3CD161/Vα24Vβ11 as an index.
2. The means for assaying the immune function according to the above 1, wherein it is used as a substitute for the ratio of T helper 1 cells/T helper 2 cells (Th1/Th2)
3. The means for assaying the immune function according to the above 1, wherein CD3CD161 and Vα24Vβ11 are assayed and the ratio thereof is calculated.
4. A method for screening a novel carcinostatic agent where CD3CD161 and Vα24Vβ11 are assayed , and the ratio thereof is in a direction of working an immune system in which CD3CD161 mainly acts is used as an index.
5. A method for assaying the immune function utilizing the means mentioned in any of the above 1 to 3.
6. A means for diagnosing the immune function utilizing the means mentioned in any of the above 1 to 3.
7. A commercial method where the means mentioned in any of the above 1 to 3 is carried on a commercial medium.
8. A business method where the means mentioned in any of the above 1 to 3 is carried on a commercial medium.

Incidentally, CD3CD161 may be sometimes mentioned as CD3 x CD161 as hereinafter.

### Brief Description of the Drawings

[Fig. 1] It shows a correlation of the ratio of CD3 x CD161/Vα24Vβ11 (TCR Vα24+/TCR Vβ11+) to the amounts of IL-12 (pg/ml) or IFNγ (IU/ml) in all cases.
[Fig. 2] It shows a correlation of the ratio of Th1/Th2 to the amounts of IL-12 (pg/ml) or IFNγ (IU/ml) in all cases.
[Fig. 3] It shows a correlation of the ratio of CD3 × CD161/Vα24Vβ11 (TCR Vα24+/TCR Vβ11+) to the amounts of IL-12 (pg/ml) or IFNγ (IU/ml) in CR, PR and long term NC.
[Fig. 4] It shows a correlation of the ratio of Th1/Th2 to the amount of IL-12 (pg/ml) or IFNγ (IU/ml) in CR, PR and long term NC.
[Fig. 5] It shows a correlation of the ratio of CD3 × CD161/Vα24Vβ11 (TCR Vα24+/TCR Vβ11+) to the amounts of IL-12 (pg/ml) or IFNγ (IU/ml) in long term NC.
[Fig. 6] It shows a correlation of the ratio of Th1/Th2 to the amounts of IL-12 (pg/ml) or IFNγ (IU/ml) in long term NC.
[Fig. 7] It shows a correlation of the ratio of CD3 × CD161/Vα24Vβ11 (TCR Vα24+/TCR Vβ11+) to the amounts of IL-12 (pg/ml) or IFNγ (IU/ml) in NC.

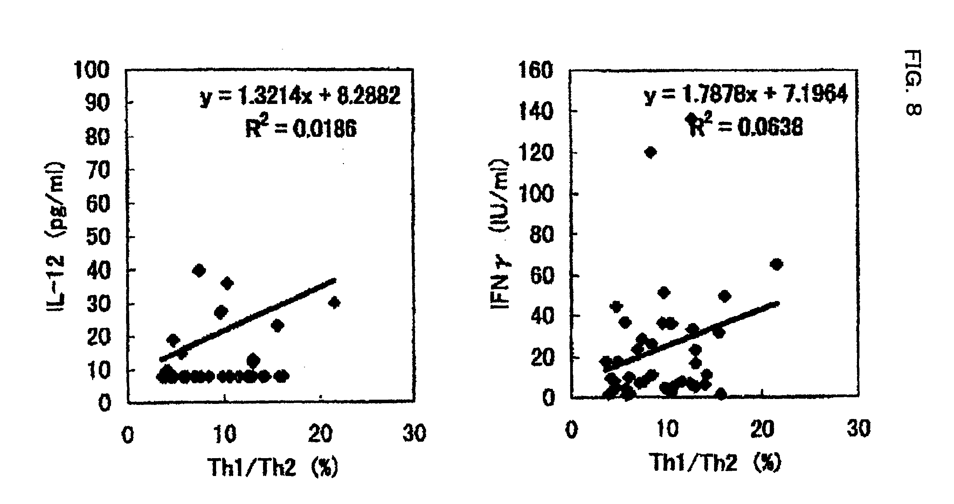

[Fig. 8] It shows a correlation of the ratio of Th1/Th2 to the amounts of IL-12 (pg/ml) or IFNγ (IU/ml) in NC.

### Best Mode for Carrying Out the Invention

As hereunder, the present invention will be illustrated in detail.

The present invention has been carried out by investigating the correlation between the clinical effect and cytokine. The present inventor has administered a substance derived from mycelia of mushroom to 70 patients suffering from cancer and various cytokines have been assayed. The results thereof have been obtained as the data showing the correlation shown by the Figures.

As shown in the Figures, the correlations of Th1/Th2 ratio to IL-12; Th1/Th2 ratio to IFNγ; CD3 × CD161/Vα24Vβ11 ratio to IFNγ; and CD3 × CD161/Vα24Vβ11 ratio to IL-12 have been tested.

Thus, in the present invention, CD3 × CD161 and Vα24Vβ11 have been assayed and it has been found that testing the ratio thereof has a correlation to inducing ability of IL-12 and producing ability of IFN-γ in the patients suffering from cancer, that the testing of the said ratio has the same significance as in the conventional Th1/Th2 ratio and that, in some canceration levels, assay of CD3 × CD161 and Vα24Vβ11 and testing of the ratio thereof are rather possible for better diagnosis.

As a result thereof, in screening a substance having an activating ability for immune function of patients suffering from cancer, it is necessary that at least the ratio of CD3 × CD161 to Vα24Vβ11 is able to select an advantageous action to CD3 × CD161 as an index. It has been also proved that, as an index for diagnosis of immune function of patients suffering from cancer, the state where immune function of active IL-12 induction and INFγ production is achieved is shown when CD3 × CD161 is dominant in the ratio of CD3 × CD161 to Vα24Vβ11 while the situation is reversed when Vα24Vβ11 is dominant.

As a result of a specific action of a substance selected by testing the ratio of CD3 × CD161 to Vα24Vβ11, production of IFNγ in a large amount is induced and, in addition, it is now possible in an immune response that immune system is induced in a direction where CD3 × CD161 of NKT cells works (becoming active) , whereupon, by the use of the said selected substance, a therapeutic agent for an extremely useful cancer immunotherapy can be provided. With regard to the selection of such a useful substance, its activating ability can be tested by, for example, checking whether it stimulates the cell holding the NKR-P1 (natural killer-P1) or, in other words, the cell having CD3 × CD161 which is a cell surface marker when the said substance is administered to living matter. The therapeutic agent selected as such may, for example, be an auxiliary food preparation for health by oral administration comprising the components derived from mycelia of mushroom.

### Clinical Examples

The present invention will now be illustrated more specifically by way of the following clinical examples.

The present inventor paid his attention to the behavior of various cytokines in the patients suffering from cancer (70 cases), assayed IFNγ, CD3 × CD161, Vα24Vβ11, IL-12, Th1/Th2 ratio, etc. and analyzed the correlations thereof to various cytokines. All patients were administered with the components of mycelia of mushroom (polysaccharides) or OK 432 (Picibanil manufactured by Chugai Pharmaceutical Co.,Ltd.) in addition thereto. All of the administered components of mycelia of mushroom (polysaccharides) were the components of mycelia of mushroom having an inducing ability for IL-12 production and an activating ability for NKT cells. Tables 1 to 5 show the amounts of each cytokine after administration of the substances derived from various kinds of mushroom (processed products of mycelia of mushroom and derived products thereof: processed product of mycelia of *Famos glaucotus* such as MAK, processed product of mycelia of *Ganoderma boninense* such as SM, processed product of *Coriolus versicolor* such as PSK, polysaccharide derived from filtrate of culture of mycelia of *Schizophyllum alneum* such as SCP and processed products of mycelia of *Cortinellus shiitake* such as AHCC and LEM) to the patients suffering various types of cancer. The dose was 3 to 6 grams per day *per os* and the term for administration was shown as "Treated Term" in the tables. Among the 70 cases, 6 cases were completely recovered (CR), 19 cases were partially recovered (PR), 25 cases were survived for 6 months or longer (being alive for 6 months or longer without progress of cancer: long term NC), 14 cases were survived for shorter than 6 months (no progress of cancer: short term NC) and 6 cases were ineffective (PD).

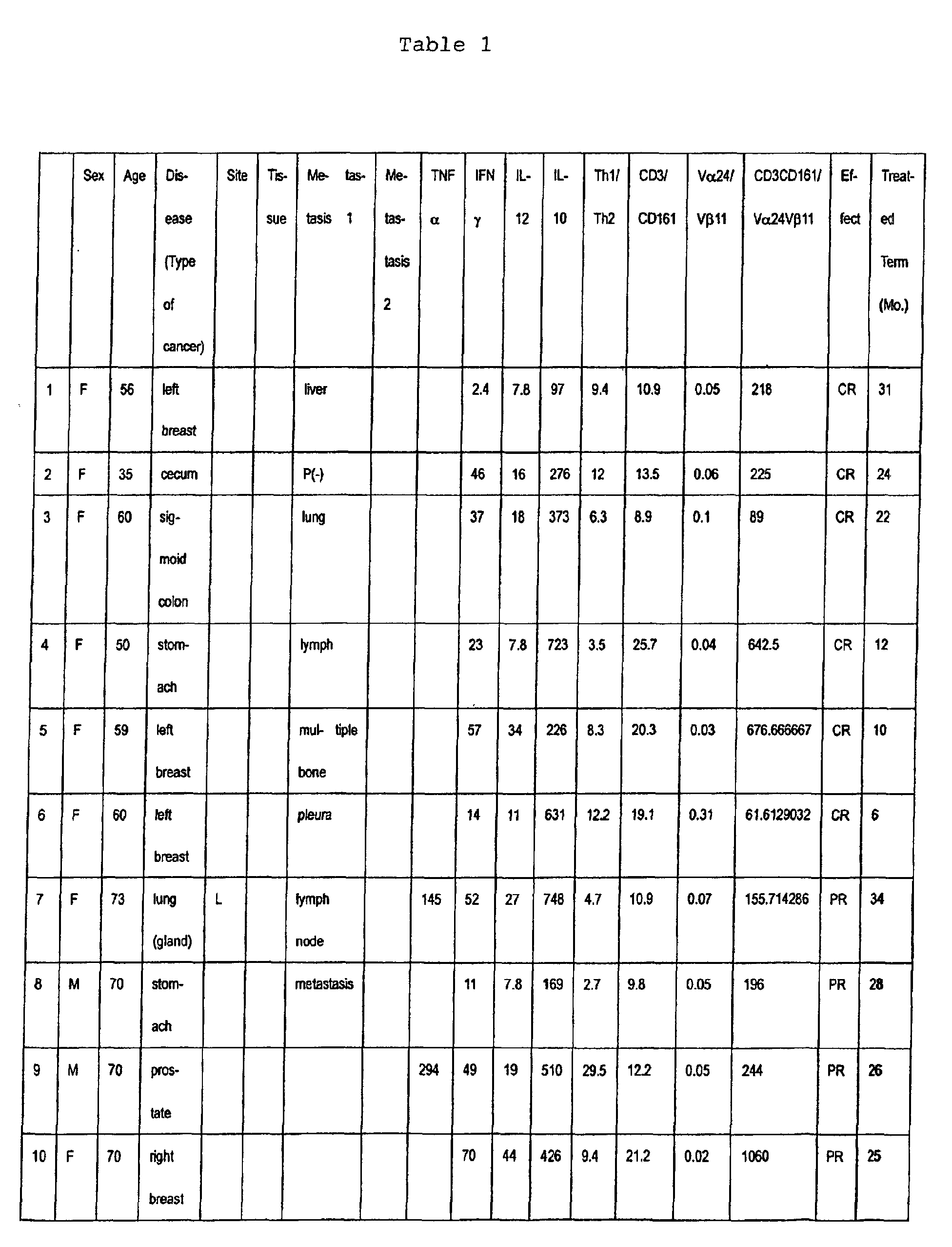

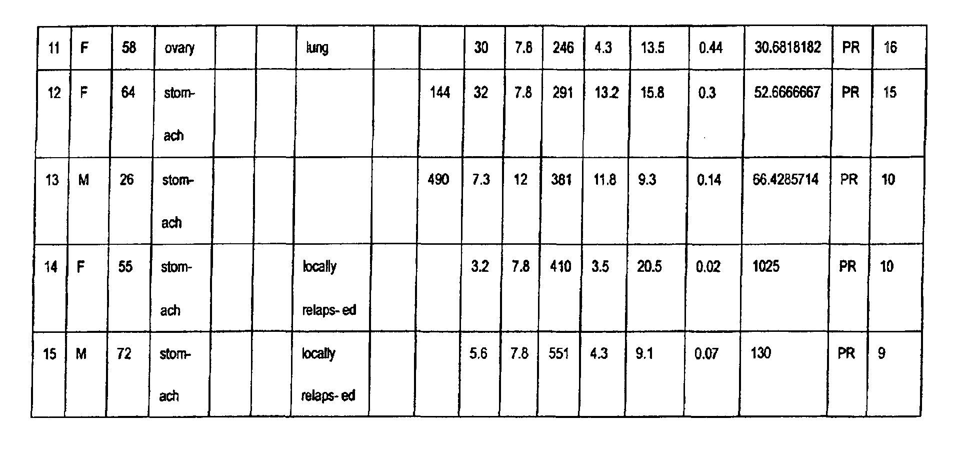

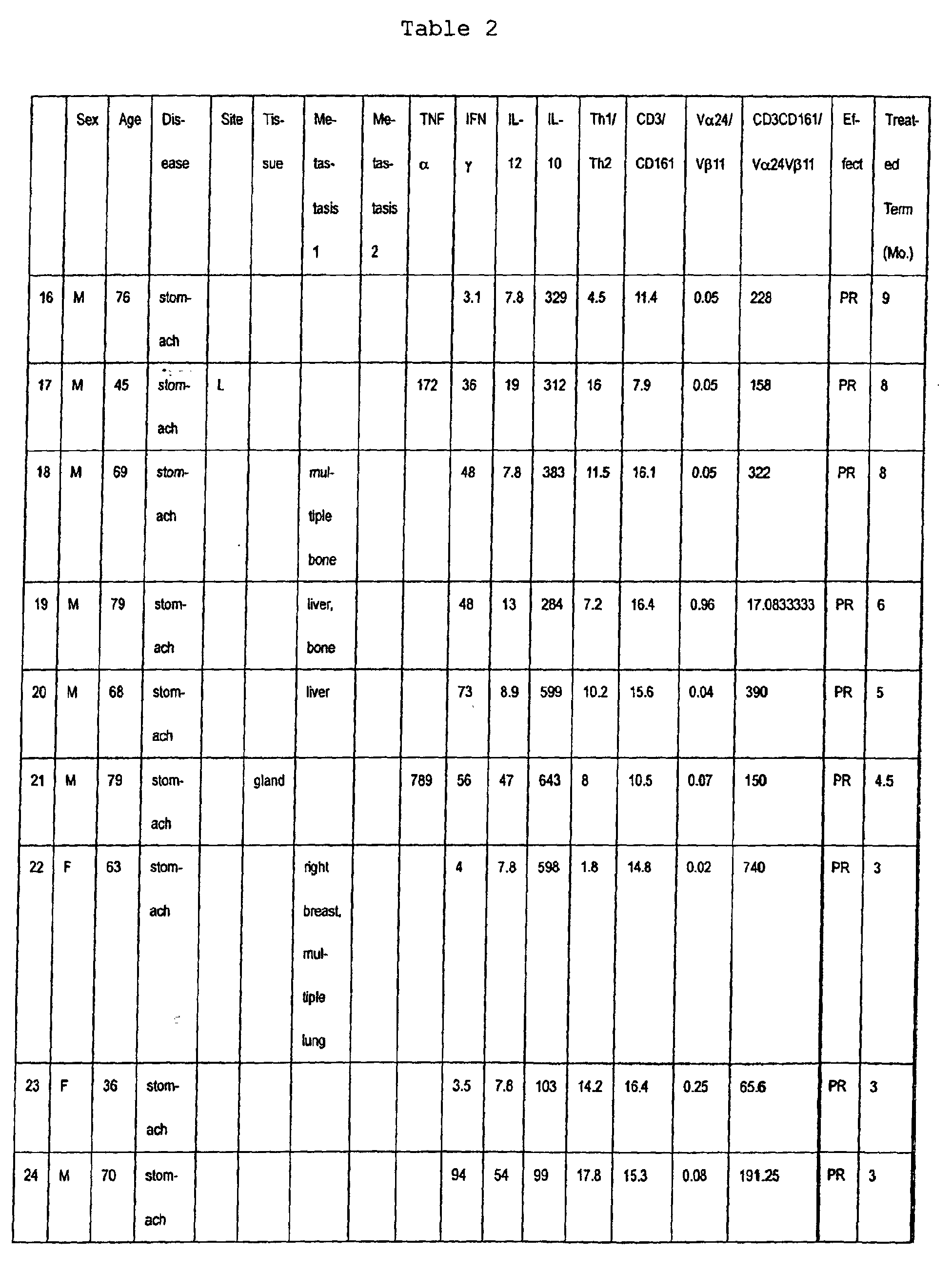

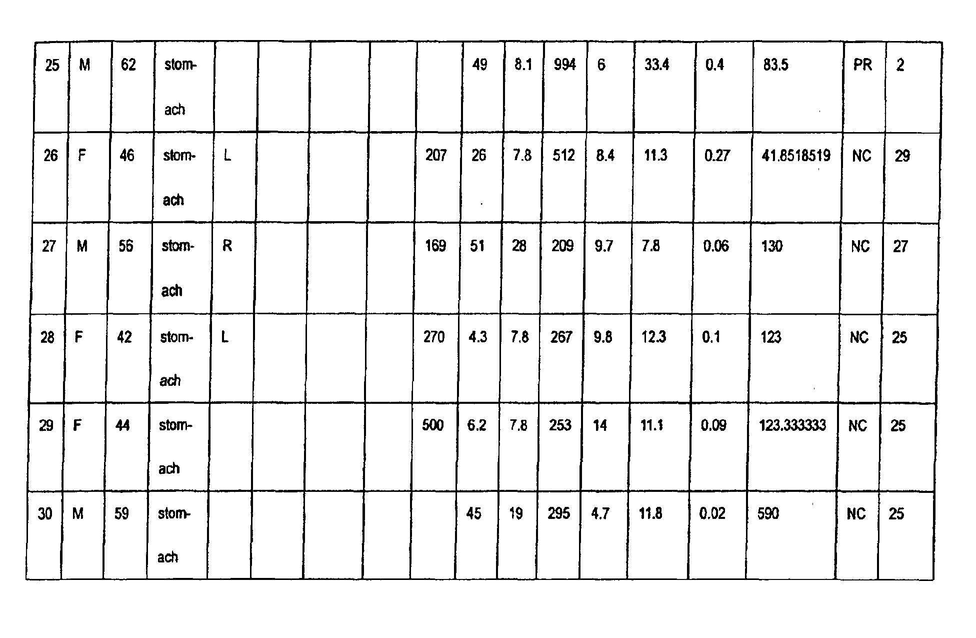

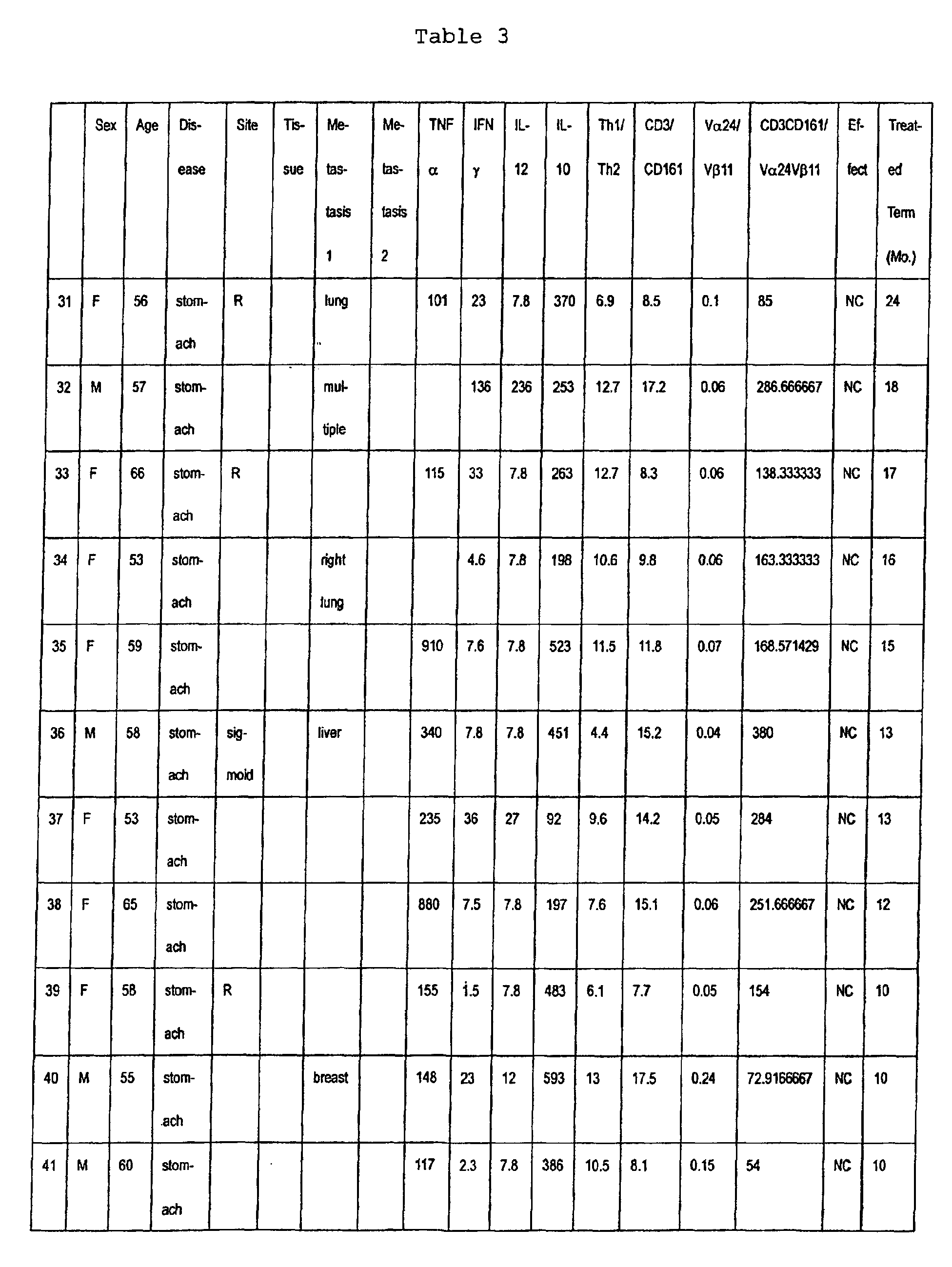

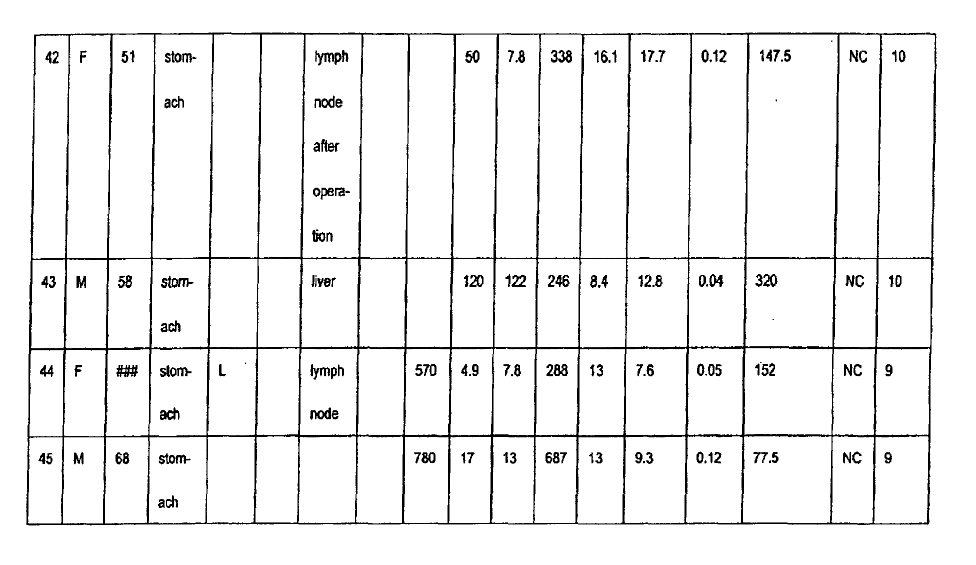

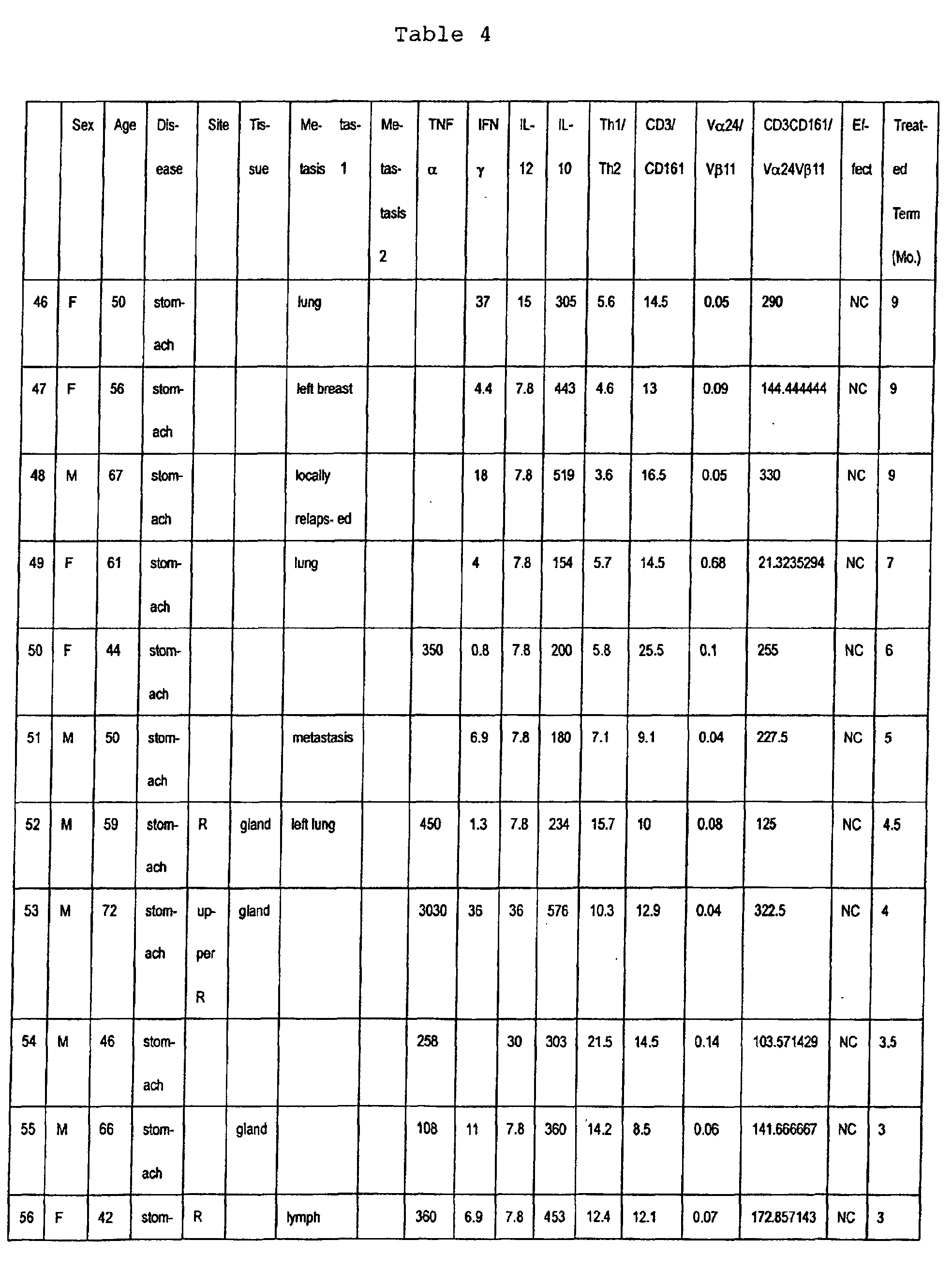

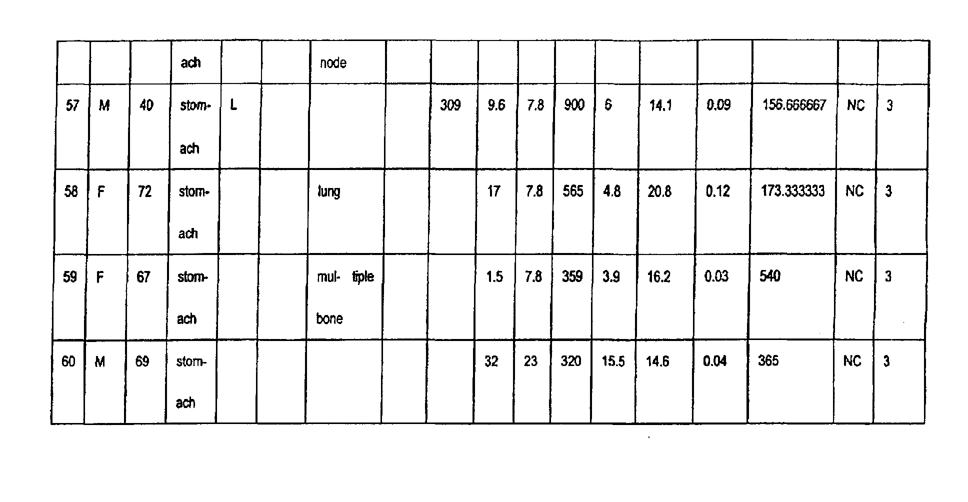

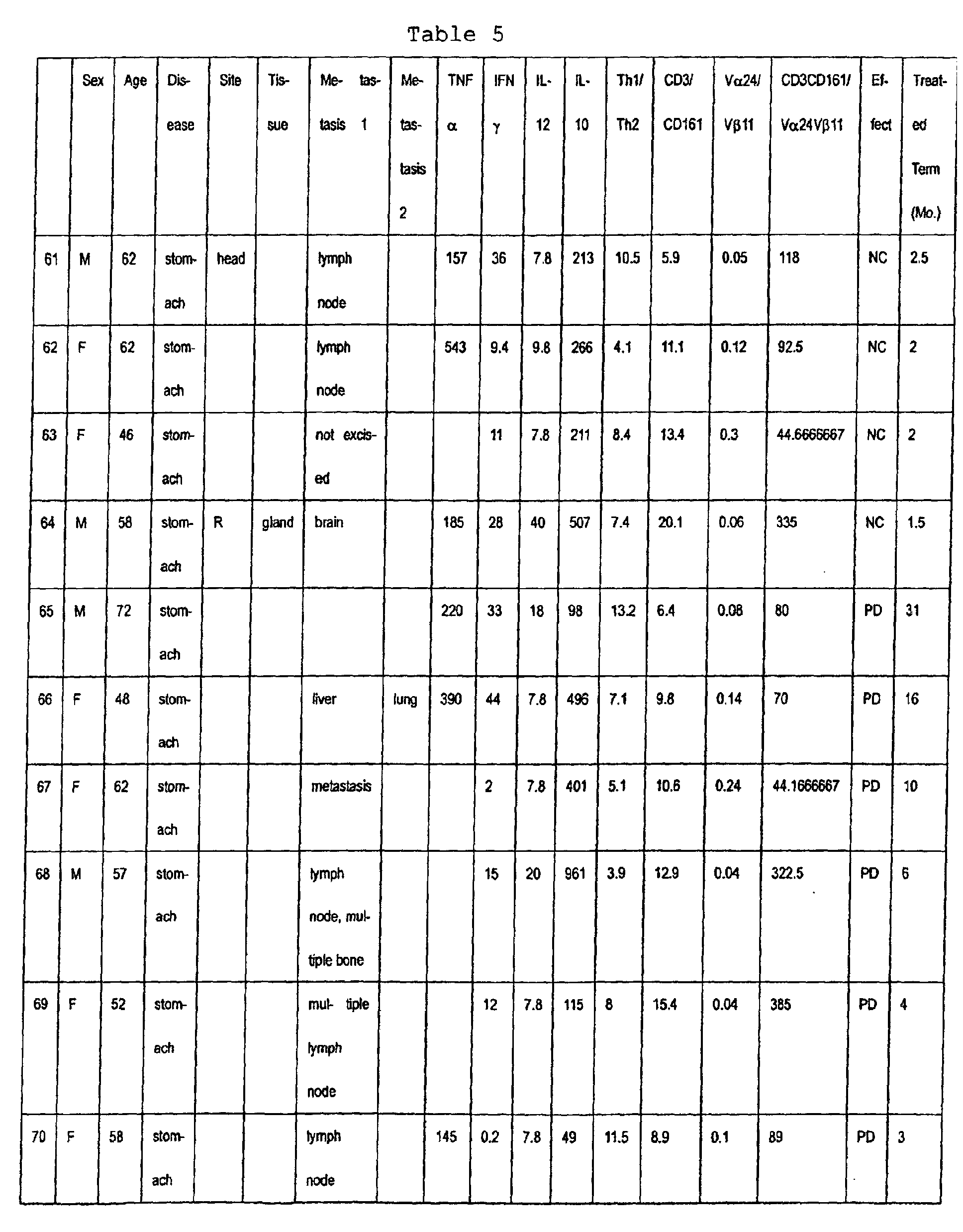

Vα24Vβ11 is also expressed as Vα24+/Vβ11+ and means a Vα24Vβ11-positive cell or a cell having Vα24 and Vβ11 which are cell surface markers on the cell surface. CD3 × CD161 is also expressed as CD3+/CD161+ and means a CD3 × CD161-positive cell or a cell having CD3 and CD161 which are cell surface markers on the cell surface. In the tables, CR shows completely cured (cancer disappeared and 4 weeks or more elapsed thereafter), PR shows partially cured (50% or more cancer was reduced), long term NC shows no reaction (growth of cancer was suppressed to an extent of 50% or less, or was suppressed to an extent of 25% or less with survival of 6 months or longer thereafter), short term NC shows no reaction (growth of cancer was suppressed to an extent of 50% or less, or was suppressed to an extent of 25% or less with survival of shorter than 6 months thereafter) and PD shows ineffective (growth of cancer was noted to an extent of 25% or more).

Methods for the assay of cells and each cytokine will be mentioned as hereunder.

### (Assay of NKT Cells)

Among activation of NKT cells, assay of activation by action to NKR-P1 can be checked an increase in NKT cell numbers by an assay of cell surface antigens (CD3 and CD161) specifically existing on the cell surface of NKT cells. To be more specific, cells which are positive to CD3 and also positive to CD161 for mono nuclear cells in peripheral blood are tested. To be more specific, cells which are positive to CD3 and also positive to CD161 are tested for mono nuclear cells in peripheral blood. Thus, CD3 and CD161 which are cell surface antigens of NKT cell are assayed by a two color test using a flow cytometry by the use of monoclonal antibody. The fact that NKT cell is activated means that, in mono nuclear cells, the ratio of NKT cells is 10% or more. Ability for activating the NKT cells means a function where the ratio of NKT cells is increased to an extent of 10% or more, or a function where further potentiation from the ratio of the NKT cells comparing to the ratio of the NKT cells in pre-administration of a certain substance taken place.

Ratio of cells where CD3 and CD161 which are cell surface antigens are positive were assayed according to a conventional manner by a two color test using a flow cytometry for cells in blood using the blood of the patient suffering from cancer. With regard to the monoclonal antibodies to CD3 and CD161, there were used CD3-PC5 manufactured by Coulter and CD161 manufactured by Becton Dickinson, respectively.

Among activation of NKT cells, assay of activation by an action to Vα24Vβ11 can be carried out by checking an increase in NKT cell numbers by the assay of cell surface antigens (Vα24 and Vβ11) specifically existing on cell surface of the NKT cells. To be more specific, cells which are positive to Vα24 and also positive to Vβ11 are tested for mono nuclear cells in peripheral blood. Thus, Vα24 and Vβ11 which are cell surface antigens on NKT cells were assayed by a two color test using a flow cytometry by the use of monoclonal antibodies (TCR-Vα24PE, TCR-Vβ11FITC; Beckman Coulter).

### (Preparation of Sample for Assay of Cytokine)

Firstly, mono nuclear cells are separated and prepared from blood of a patient suffering from cancer. Heparin-added peripheral blood obtained from the patient suffering from cancer was diluted to an extent of two-fold using a phosphate buffer saline (PBS), mixed, layered on a Ficoll-Conray solution (specific gravity: 1.077) and centrifuged at 400G for 20 minutes to collect a mono nuclear cell layer. After washing, an RPMI-1640 medium to which 10% fetal bovine serum (FBS) were added was added thereto so as to make the mono nuclear numbers 1 x 10⁶. To 200 µl of the cell suspension was added phytohemagglutinin (hereinafter, abbreviated as PHA) (manufactured by DIFCO) to make its concentration 20 µg/ml followed by incubating at 37°C for 24 hours on a 96-well microplate in the presence of 5% of CO₂ to prepare a sample for the assay of cytokine in the said incubated cell solution.

### (Assay of IL-12)

IL-12 was assayed by an ELISA method using a kit manufactured by R & D Systems. Practically, each 50 µl of an assay diluent RD1F and 200 µl of the standard or a sample prepared in Example 1 were placed in each well of a 96-well microplate and made to react by being allowed to stand at room temperature for 2 hours. After that, each 200 µl of anti-IL-12 antibody labeled with horse radish peroxidase (hereinafter, abbreviated as HRP) were placed followed by being allowed to stand at room temperature for 2 hours. The reaction solution in each of the wells was taken out, washed for three times, each 200 µl of coloration substrate solution were placed followed by being allowed to stand at room temperature for 20 minutes and each 50 µl of a solution for stopping the enzyme reaction were placed. Absorbance at 450 nm of each well was measured using 550 nm as a control in terms of Emax (Wako Pure Chemicals). Amount of IL-12 is expressed in pg/ml. Ability for inducing the production of IL-12 means a function where the amount of IL-12 produced by stimulation of peripheral blood mono nuclear cells is potentiated to an extent of 7.8 pg/ml or more (7.8 being an assay limit), or a function where potentiation of the amount of IL-12 production comparing to an amount of IL-12 production in pre-administration of a certain substance taken place.

### (Assay of IFNγ)

Assay of IFNγ was carried out by an enzyme immunoassay (EIA) using an IFNγ EASIA kit of BioSource Europe S. Practically, each 50 µl of the standard or the above-prepared sample diluted to an extent of two-fold were placed in each well of a 96-well microplate, each 50 µl of anti IFN-γ antibody labeled with HRP were placed and the reaction was carried out at room temperature for 2 hours with shaking. The reaction solution in each well was taken out and washed for three times, each 200 µl of a coloration substrate solution were placed, the reaction was carried out with shaking for 15 minutes at room temperature and each 50 µl of a solution for stopping the enzyme reaction were placed. Absorbances at 450 nm and 490 nm in each well were measured using 630 nm as a control in terms of Emax (Wako Pure Chemical). Amount of IFNγ is expressed in IU/ml.

### (Assay of Th1/Th2 Cell Ratio)

The Th1/Th2 cell ratio was tested according to a conventional method using "a helper T (Th) cell strain three color analysis test" by means of a flow cytometry. The Th1/Th2 means the ratio of cells (Th1) producing IFNγ to cells (Th2) producing IL-4 among the helper T cells having CD4 which is a cell surface antigen and is mentioned as CD4 × IFNγ/IL-4.

Firstly, blood from a patient suffering from cancer was treated at 37°C for 4 hours with phorbol 12-myristate 13-acetate and ionomycin so that the cells in the blood were stimulated to generate cytokine. Then breferdin A was added to stop the generation reaction, CD4 which is a cell surface marker was stained with CD4-PC5 (Beckman Coulter) which is an anti-CD4 antibody to fix the cells and a hemolytic treatment was carried out using an FACS lysing solution (Nippon Becton Dickinson). After that, a cell membrane permeation treatment was carried out by an FACS permeabilizing solution (Nippon Becton Dickinson), then cytokine in the cells was stained with anti-IFNγ antibody/anti-IL-4 antibody (Fastimmune IFNγ FITC/IL-4 PE; Nippon Becton Dickinson) and assay and analysis were carried out using a flow cytometer (FACS calibur; Becton Dickinson).

On the basis of the data which are the result of the above assay, correlation of each cytokine was analyzed as follows.

### [Test Example 1]

### (Results in All 70 Cases)

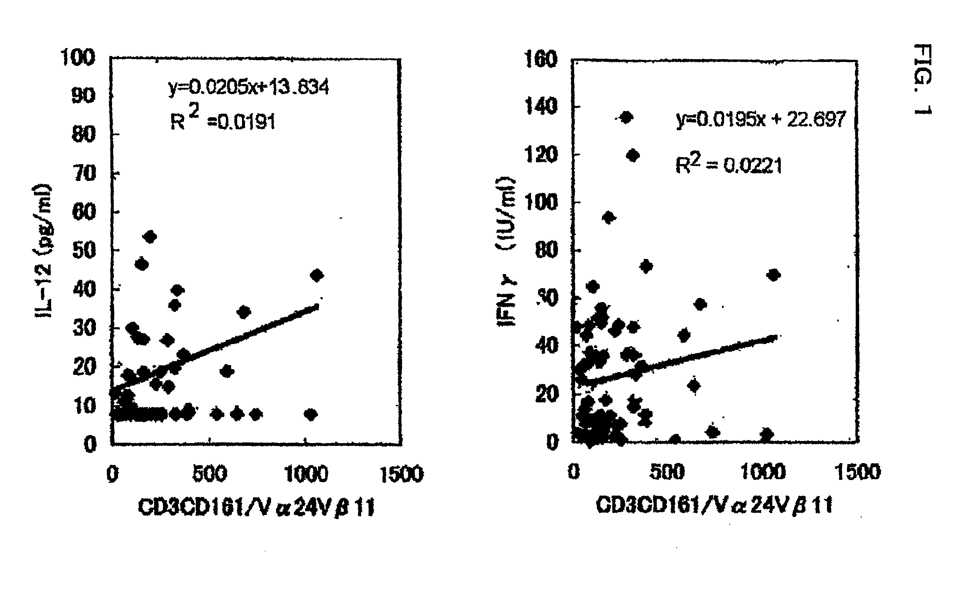

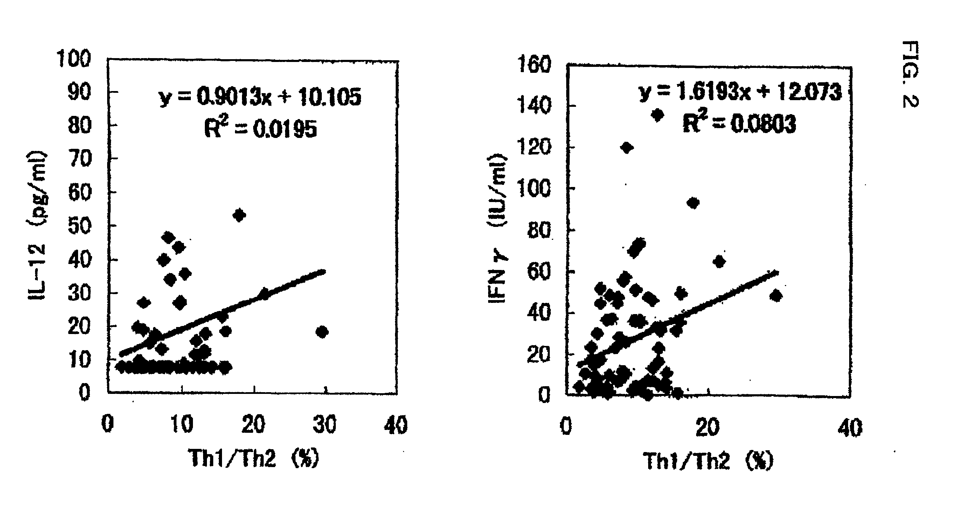

Fig. 1 shows the correlation of CD3 × CD161/Vα24Vβ11 (TCR Vα24+/TCR Vβ11+) ratio to IL-12 (pg/ml) and IFNγ (IU/ml) amounts in all 70 cases. Fig. 2 shows the correlation of Th1/Th2 ratio to IL-12 (pg/ml) and IFNγ (IU/ml) amounts in all 70 cases.

As a result, it was found that the Th1/Th2 ratio and the CD3 × CD161/Vα24Vβ11 ratio showed nearly the same tendency and that, when Th1 and CD3 × CD161 were dominant, there was a tendency of induction of IL-12 and production of IFNγ. Accordingly, it was confirmed that the test for the CD3 × CD161/Vα24Vβ11 ratio was useful for the test of immune function in a patient suffering from cancer.

### [Test Example 2]

### (Result in 50 Cases of CR, PR and Long Term NC)

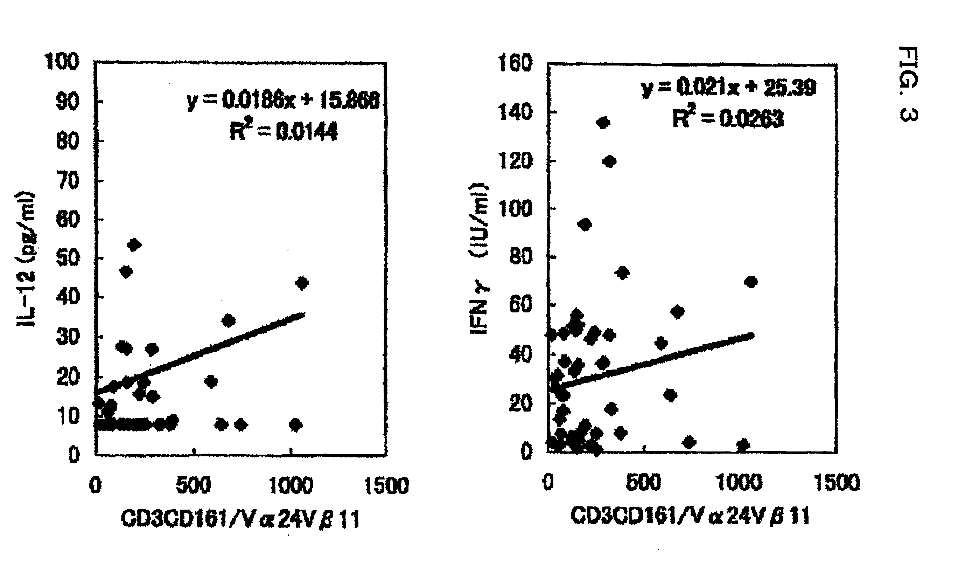

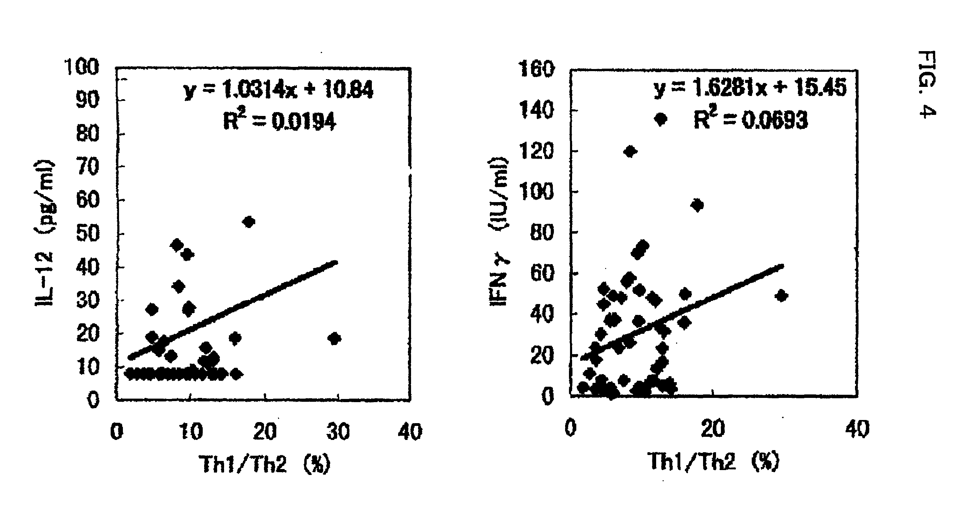

Fig. 3 shows the correlation of CD3 × CD161/Vα24Vβ11 (TCR Vα24+/TCRVβ11+) ratio to IL-12 (pg/ml) and IFNγ (IU/ml) amounts in the selected 50 cases. Fig. 4 shows the correlation of Th1/Th2 ratio to IL-12 (pg/ml) and IFNγ (IU/ml) amounts in the selected 50 cases.

As a result, it was found that the Th1/Th2 ratio and the CD3 × CD161/Vα24Vβ11 ratio showed nearly the same tendency and that, when Th1 and CD3 × CD161 were dominant, there was a tendency of induction of IL-12 and production of IFNγ. Accordingly, it was confirmed that the test for the CD3 × CD161/Vα24Vβ11 ratio was useful for the test of immune function in a patient suffering from cancer.

### [Test Example 3]

### (Result in 25 Cases of Long Term NC)

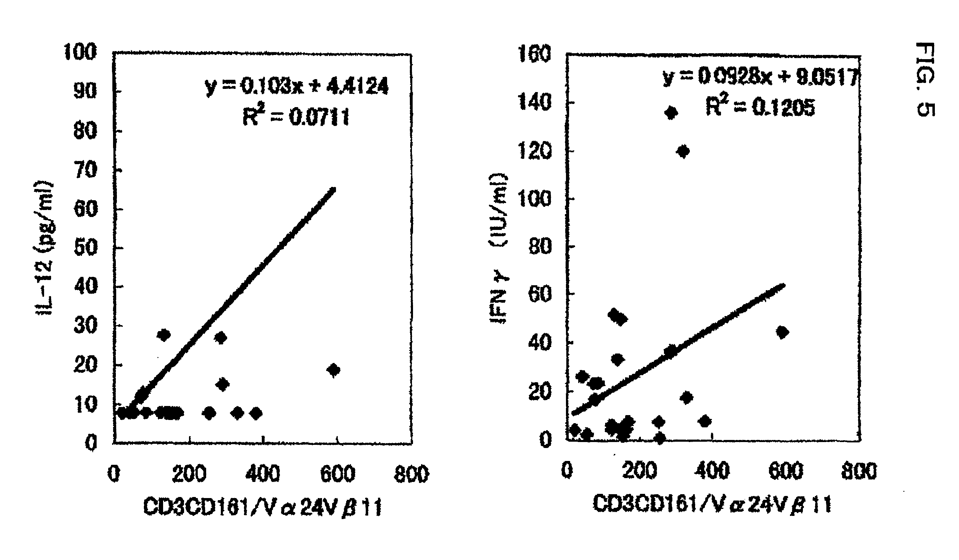

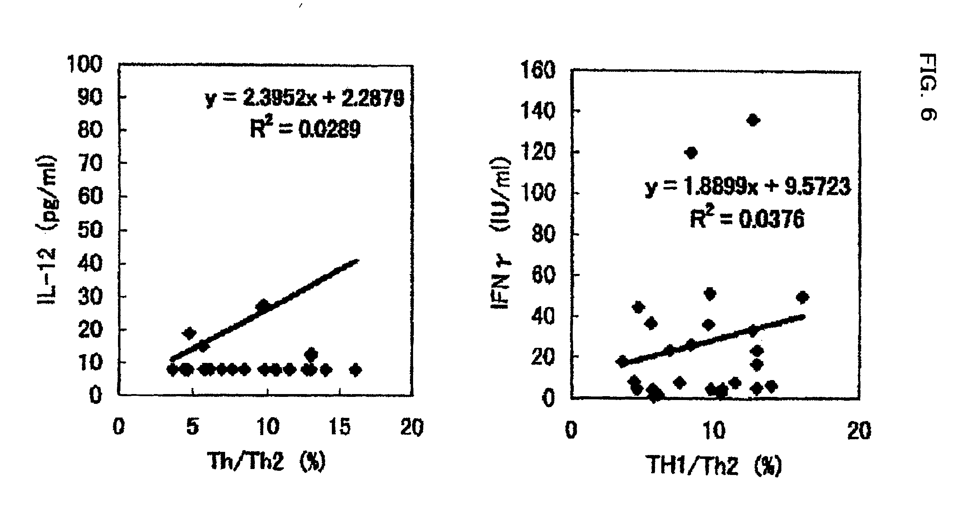

Fig. 5 shows the correlation of CD3 × CD161/Vα24Vβ11 (TCR Vα24+/TCR Vβ11+) ratio to IL-12 (pg/ml) and IFNγ (IU/ml) amounts in the selected 25 cases. Fig. 6 shows the correlation of Th1/Th2 ratio to IL-12 (pg/ml) and IFNγ (IU/ml) amounts in the selected 25 cases.

As a result, it was found that the Th1/Th2 ratio and the CD3 × CD161/Vα24Vβ11 ratio showed nearly the same tendency or showed that the latter was clearer tendency and that, when Th1 and CD3 × CD161 were dominant, there was a tendency of induction of IL-12 and production of IFNγ. Accordingly, it was confirmed that the test for the CD3 × CD161/Vα24Vβ11 ratio was useful for the test of immune function in a patient suffering from long term NC cancer.

### [Test Example 4]

### (Result in 39 Cases of NC)

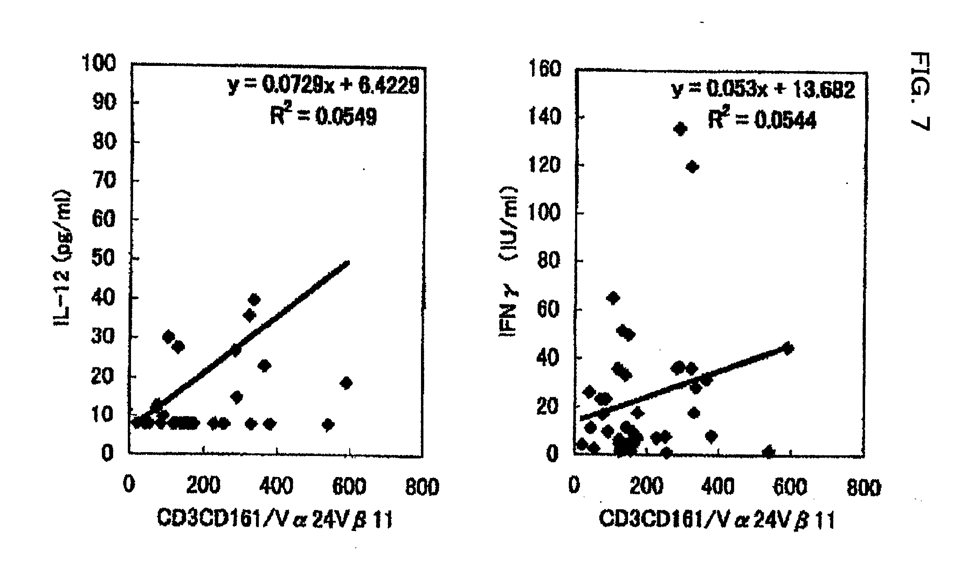

Fig. 7 shows the correlation of CD3 × CD161/Vα24Vβ11 (TCR Vα24+/TCR Vβ11+) ratio to IL-12 (pg/ml) and IFNγ (IU/ml) amounts in the selected 39 cases. Fig. 7 shows the correlation of Th1/Th2 ratio to IL-12 (pg/ml) and IFNγ (IU/ml) amounts in the selected 39 cases.

As a result, it was found that the Th1/Th2 ratio and the CD3 × CD161/Vα24Vβ11 ratio showed nearly the same tendency or showed that the latter was clearer tendency and that, when Th1 and CD3 × CD161 were dominant, there was a tendency of induction of IL-12 and production of IFNγ. Accordingly, it was confirmed that the test for the CD3 × CD161/Vα24Vβ11 ratio was useful for the test of immune function in a patient suffering from NC cancer.

### Advantages of the Invention

On the basis of a finding that, in a cascade in which activated NKT cells bearing cancer immune are participated, function and merit of the NKT cells to antigen receptors are entirely different between NKR-P1 (natural killer-P1) and Vα24Vβ11, the present invention provides a novel assay means for immune function where CD3CD161 and Vα24Vβ11 are measured and the ratio thereof is used as an index.

## Claims

1. A means for assaying an immune function using the ratio of CD3CD161/Vα24Vβ11 as an index.

2. The means for assaying the immune function according to claim 1, wherein it is used as a substitute for the ratio of T helper 1 cells/T helper 2 cells (Th1/Th2).

3. The means for assaying the immune function according to claim 1, wherein CD3CD161 and Vα24Vβ11 are assayed and the ratio thereof is calculated.

4. A method for screening a novel carcinostatic agent where CD3CD161 and Vα24Vβ11 are assayed and the fact that the ratio thereof is in a direction of working an immune system in which CD3CD161 mainly acts is used as an index.

5. A method for assaying the immune function utilizing the means mentioned in any of claims 1 to 3.

6. A means for diagnosing the immune function utilizing the means mentioned in any of claims 1 to 3.

7. A commercial method where the means mentioned in any of claims 1 to 3 is carried on a commercial medium.

8. A business method where the means mentioned in any of claims 1 to 3 is carried on a commercial medium.
